# EUROPEAN PATENT APPLICATION

(11) **EP 4 613 373 A1**
(43) Date of publication of application: **10.09.2025**
(21) Application number: 24161530.1
(22) Date of filing: 05.03.2024
(51) Int. Cl.: B01J 23/644, C07C 45/39

(54) **METHOD FOR THE CATALYTIC PREPARATION OF 1,3-DIHYDROXYACETONE**

(71) Applicant: Universität Ulm, 89081 Ulm (DE); Institute of Chemical Process Fundamentals of the CAS, v. v. i., 16500 Prague 6 (CZ)
(72) Inventor: VILK, Yury, 89075 Ulm (DE); DYTRYCH, Pavel, 14900 Praha 4 (CZ); BERANEK, Radim, 89081 Ulm (DE)
(74) Representative: Pfenning, Meinig & Partner mbB

(57) **Abstract**

The invention describes a method for the preparation of 1,3-dihydroxyacetone (DHA) comprising the selective catalytic conversion of glycerol in the presence of a catalytic amount of a heterogeneous catalyst comprising or consisting of a composite of a metal cluster and/or a metal oxide cluster and a metal chalcogenide semiconductor powder, an antimony-containing powder and/or a lead-containing powder or pluralities of the aforementioned compounds and one or more oxidizing agents, characterized in that the catalytic reaction is carried out in the absence of UV and visible light and the reaction is not conducted electrochemically.

## Description

The present invention relates to a catalytic method for the production of 1,3-dihydroxyacetone starting from glycerol.

1,3-Dihydroxyacetone (DHA) is currently used primarily in the cosmetics industry and also has great potential for use in the synthesis of new biodegradable polymers if the market price (approx. US$ 150/kg) were lower. Glycerol is a starting chemical that is very inexpensive (market price approx. US$ 0.6/kg) and at the same time renewable, as it is a waste product of biodiesel production. The development of new chemical processes for the utilization of glycerol is therefore of great importance, whereby the selective conversion of glycerol to DHA is one of the most attractive strategies.

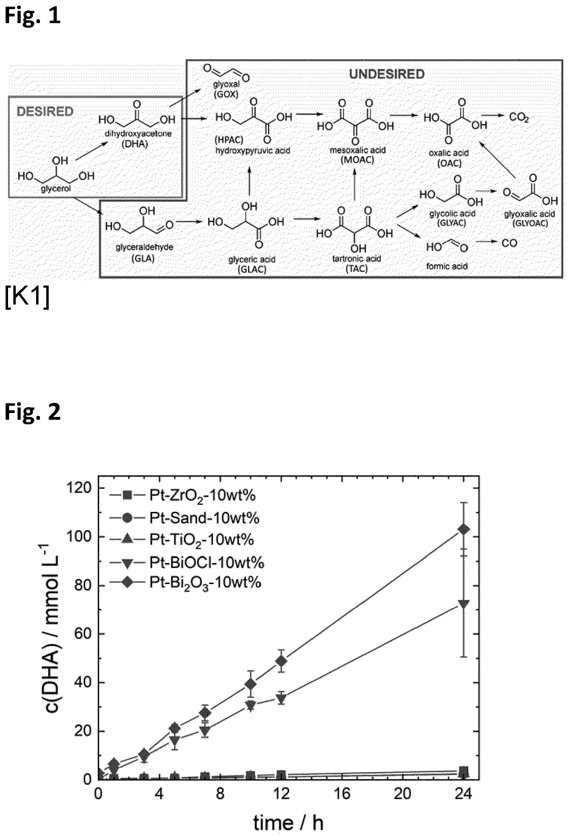

From a chemical point of view, the greatest challenge in the production of DHA from glycerol is the selective oxidation of the secondary hydroxyl group of glycerol, whereby the oxidation of the usually more reactive primary hydroxyl groups should be avoided, as well as all subsequent reactions of the DHA formed (Fig. 1).

Ideally, the conversion of glycerol to DHA should be catalytic, under normal conditions (room temperature and normal pressure), using readily available oxidizing agents (atmospheric oxygen or water) and without any additional costly energy input (e.g. electrical energy).

Various processes for the selective oxidation of glycerol to DHA are known. On an industrial scale, DHA is nowadays obtained by the microbial fermentation of glycerol by, for example, Gluconobacter suboxydans or Acetobacter suboxydans to dihydroxyacetone (L. A. Underkofler et al., J. Am. Chem. Soc., 59, 301-302 (1937); H.-L. Ohrem (Merck Patent GmbH), DE-A-4341238 (1993); H.-L. Ohrem (Merck Patent GmbH), DE-A-4444404 (1994); R. Bauer, dissertation, Technical University of Munich (2005)). Although these microbial enzymatic reactions allow high selectivity of the conversion, they are very costly, as only low concentrations of glycerol can be used, which leads to unfavorable space-time yields. The development of alternative catalytic processes is therefore highly desirable.

Various homogeneous catalysts have been investigated for this reaction. Iron salts with hydrogen peroxide solution as oxidizing agent (Fenton reagent) can oxidize glycerol to DHA. However, a greatly increased formation of by-products such as glycolic acid and glyceraldehyde can be observed (V. Maksimovic et al., Ann. N. Y. Acad. Sci., 1048, 461-465 (2005); V. Maksimovic et al., Carbohydr. Res., 341, 1828-1833 (2006); V. Laurie et al., J. Agric. Food. Chem., 54, 4668-4673 (2006)). Some palladium complexes (with benzoquinone as oxidizing agent) catalyze the conversion of glycerol to DHA with high selectivity, but exhibit low stability and lower selectivity when atmospheric oxygen is used as oxidizing agent (R.M. Painter et al., R. M. Angew. Chem., Int. ed., 49, 9456 (2010)). Recently, a three-step process was also presented in which acetylation of glycerol with benzaldehyde is first performed at 80 °C, then the product of acetylation is oxidized in the presence of 2,2,6,6-tetramethylpiperidinyloxyl (TEMPO) with NaClO as oxidant at 65 °C, and then DHA and benzaldehyde are obtained by the hydrolysis of the oxidation product (Z. Zheng et al., Ind. Eng. Chem. Res., 51, 3715 (2012)).

In terms of industrial application, heterogeneous catalysts are much more favorable and desirable. The heterogeneous catalysts studied so far were usually carbon-supported monometallic (Pt, Pd, Au) (S. Demirel et al., Appl. Catal. B: Environ, 70, 637 (2007); S. Carrettin et al., J. Phys. Chem. Chem. Phys., 5, 1329 (2003)) or bimetallic (Pt-Au (S. Demirel et al., Appl. Catal. B: Environ., 70, 637 (2007)); Pd-Ag (S. Hirasawa et al., Catal. Sci. Technol., 2, 1150 (2012)); Pt-Bi (H. Kimura et al., Appl. Catal. A: Gen., 96, 217 (1993); H. Kimura, Appl. Catal. A: Gen., 105, 147 (1993); P. Gallezot, Catal. Today, 37, 405 (1997); W. Hu et al, Ind. Eng. Chem. Res., 49, 10876 (2010); N. Worz et al., J. Phys. Chem. C, 114, 1164 (2010) and A. Brandner, Dissertation, Technische Universität Darmstadt (2009)) catalysts. However, numerous by-products are formed in high yields due to the oxidation of the primary alcohol group. The selectivity here is only in the range of 20 to 40 %. The most promising results to date have been reported for Pt-Bi catalysts, with the following benchmark values: DHA yield of 48% and glycerol conversion of 80% at 80 °C (W. Hu et al., Ind. Eng. Chem. Res., 49, 10876 (2010)).

The electrochemical conversion of glycerol to DHA at room temperature is also known. On the one hand, this involves anodic oxidation of glycerol on a glassy carbon electrode in the presence of TEMPO as a catalyst (R. Ciriminna et al., Tetrahedron Lett., 47, 6993 (2006)). This method provides only low yields (~25 %), similar to the laborious and costly enzymatic process. On the other hand, an electrochemical conversion of glycerol to DHA was reported in which very high selectivity (almost 100 %) was achieved using a carbon-supported platinum electrode saturated at the surface with Bi<3+> ions (Y. Kwon et al., ACS Catalysis, 2, 759 (2012)).

In addition, bismuth oxide-supported platinum catalysts are known to be obtainable by photoreduction of Bi₂O₃-containing H₂PtCl₆ solutions with UV light (λ > 300 nm) and are suitable for decomposition of organic environmental toxins/wastes by visible light (R. Li et al, Green Chem., 12, 212-215 (2010)). The use of such catalysts for the aerobic oxidation of n-butanol has also been described (T. Lu et al., Green Chem., 15, 2215-2221 (2013)).

Since the process used on an industrial scale (microbial fermentation) is very complex with regard to the reaction conditions (inhibition of bacterial growth by the reactant glycerol as well as by the product DHA), which leads to very unfavorable space-time yields and high costs, and the catalytic processes known to date do not yet provide satisfactory yields or DHA contaminated by by-products, the task of the present invention was to provide an improved production process for DHA.

The present invention is based on the task of the heterogeneous catalytic conversion of glycerol to dihydroxyacetone at room temperature and using air as an oxidizing agent. The catalyst used ensures a very high selectivity of the desired product and enables an uncomplicated upscaling of the technical process through the simple synthesis of the catalyst itself. The invention proposed here thus represents a practicable alternative to the established production method. Ideally, the conversion of glycerol to DHA should take place catalytically, under normal conditions (room temperature and normal pressure), using readily available oxidizing agents (atmospheric oxygen or water) and without any additional costly energy input.

This task is solved with the features of patent claim 1. The dependent claims describe preferred embodiments of the invention.

The present invention thus relates to a process for the preparation of 1,3-dihydroxyacetone (DHA) comprising the selective catalytic conversion of glycerol in the presence of
a) a catalytic amount of a heterogeneous catalyst comprising or consisting of a composite of a metal cluster and/or a metal oxide cluster and a metal chalcogenide powder, an antimony-containing powder and/or a lead-containing powder or pluralities of the aforementioned compounds and
b) one or more oxidizing agents.

According to the invention, the catalytic reaction is carried out in the absence of UV and visible light (i.e. in the dark without any need for irradiation sources or photon-utilizing photoreactors). In addition, the catalytic conversion is not carried out electrochemically, i.e. no electrical current is applied to the reaction mixture via electrodes.

The catalyst has two components: i) metal chalcogenide (which comprises metal oxides, metal tellurides, metal sulfides, etc.) powder that acts as a support and ii) metal (e.g. Pt) clusters (i.e. very small nanoparticles) which are present on the surface of the metal chalcogenide powder support. These clusters can be often covered by a thin layer of oxide, or can even be actually fully in form of oxides. These clusters also can be referred to as "metal or oxidized metal clusters.

Quite surprisingly, it was found that the catalytic conversion of glycerol, i.e. the selective oxidation, can also be carried out in the absence of external energy sources, such as UV lamps or electrochemical energy input. Surprisingly, also a very high selectivity towards DHA of >90% is achieved. In comparison with the process disclosed in DE102014212900A1, surprisingly, comparable yields and reaction rates were observed.

The process according to the invention offers the following highly relevant advantages:
- It enables the conversion of high glycerol concentrations, which leads to a better space-time yield.
- The catalytic conversion can use water and atmospheric oxygen as readily available oxidizing agents.
- When water is used as an oxidizing agent, hydrogen is obtained as a by-product.
- The catalysts used are simple and inexpensive to produce.
- As this is a heterogeneous catalytic reaction, product recovery is much simpler compared to homogeneous catalytic processes.

Further, the inventive method can be very easily scaled up since it operates at room temperature with no additional energy input, i.e. it does not require any additional heat, photon or electric current management. Also long reaction times for e.g. more than 850 hours have been demonstrated to not compromise the observed high selectivity. Thus, the process enables the formation of amounts of DHA that are high enough to guarantee formation of biodegradable polymers upon simple addition of conventional reagents.

The method according to the present invention preferably can be performed in a single batch manner.

To increase the conversion efficiency and selectivity, the catalyst composite preferably contains metal (e.g. of Pt, Au, Ag, Ru, Rh, Pd, Cu, Co, Mn, Ni) or one or more metal oxide clusters containing at least one of the aforementioned metals in addition to the metal chalcogenide powder.

Preferably, the active heterogenous catalyst used for the selective catalytic oxidation of glycerol to DHA is based on a semiconductor metal chalcogenide powder, such as semiconductor metal oxides, semiconductor metal sulfides and semiconductor metal tellurides, preferably from a bismuth-containing powder (e.g. of Bi₂O₃, Bi₂S₃, Bi₂Te₃, Bi₂Se₃, BiOCI, BiOBr, BiOI, BiVO₄ and Bi₂MoO₆). Bi₂O₃ is particularly preferred. Bi₂O₃ which is suitable for the inventive method can preferably be produced by calcination of (BiO)₂CO₃, but it can also be produced in a different way, e.g. by calcination of BiONOs, Bi(NO₃) · 5 H₂O, or BiOCI. Alternatively or in addition the heterogenous catalyst can comprise or consist of a antimony-containing powder and/or a lead-containing powder or pluralities of the aforementioned compounds.

Preferably, the surface of the bismuth-containing powder is further modified with metal (or metal oxide) clusters. Such clusters can be produced on the surface of the bismuth-containing compound, e.g. by deposition precipitation in the presence of a suitable precursor. For example, small platinum clusters can be deposited on the surface of semiconducting Bi₂O₃ when H₂PtCl₆ (as platinum precursor) is added to a suspension of Bi₂O₃ and the suspension is exposed to light (in the presence of a reducing agent). In a particularly preferred embodiment of the invention, the catalyst is Bi₂O₃ coated with Pt.

In addition, suitable catalyst composites can be obtained by colloidal deposition of metal (Pt) particles on Bi₂O₃, impregnation of Bi₂O₃ with precursors, mixing of Bi₂O₃ and metal (Pt) particles.

The amount of metal (or metal oxide) in the catalyst is preferably from 0.01 to 90 % by weight, preferably from 1 to 10% by weight, based on the total weight of the catalyst. In the process according to the invention, a catalyst quantity of 0.01 to 100 g per kg, particularly preferably 0.5 to 5 g per kg of glycerol to be converted is preferably used.

According to a further preferred embodiment, the oxidizing agent is selected from water, air (atmospheric oxygen), hydrogen peroxide. Particularly preferred is the use of water and/or air, especially air.

In the event that water is used as the oxidizing agent, hydrogen can be obtained as a by-product. In this case, a weight ratio of glycerol to water of 1:100 to 10:1 is preferably used.

The reaction can be carried out in particular at temperatures between 10 and 90 °C, preferably between 15 and 60 °C, particularly preferably between 20 and 40 °C. The invention therefore can be carried out at room temperature without the need of any input of thermal energy. With other words, no heating is necessary.

The process according to the invention can be carried out continuously or in batch mode, with the reaction time preferably being at least 1 hour.

The present invention is described in more detail with reference to the following embodiments, without limiting the invention to the embodiments shown.

The catalyst preparation was performed with the following method steps:
1) basic bismuth nitrate (solid) - main catalyst component, mass depends on the catalyst preparation batch. 20% mass loss during the calcination step
2) H₂PtCl₆*6H₂O (solid) - second main catalyst component, required mass 21% of the basic bismuth nitrate
3) Methanol (liquid) - 5mL/1.2 g (basic bismuth nitrate)
4) Water (liquid) - 5mL /1.2 g (basic bismuth nitrate)

The glycerol oxidation was conducted with the following components and conditions:
1) Pt-Bi₂O₃-10wt% (catalyst) - 16 g/L
2) Glycerol - 10% v/v (in water)
3) Diluted hydrochloric acid to set up pH to 1.8. During the process only analytic samples were taken, keeping the initial ratio of catalyst and starting reaction solution constant.

The figures show:
Fig. 1 a schematic representation of the possible reaction pathways in the oxidation of glycerol,
Fig. 2 the time course of the concentration of DHA in glycerol oxidation,
Fig. 3 the time course of the selectivity of DHA (concentration of DHA per sum of the concentrations of all oxidation products) of a glycerol oxidation,
Fig. 4 the time course of the performance of DHA production (concentration of DHA per time) of a glycerol oxidation,
Fig. 5 the time course of the selectivity of DHA (concentration of DHA per sum of the concentrations of all oxidation products) of a glycerol oxidation,
Fig. 6 the time course of the selectivity of DHA (concentration of DHA per sum of the concentrations of all oxidation products) of a glycerol oxidation,
Fig. 7 the time course of the performance of DHA production (concentration of DHA per time) of a glycerol oxidation.

To prove the effectiveness of the invention, laboratory experiments were carried out which included the production of the catalyst (up to two-digit gram range) as well as the evaluation of the heterogeneous catalysis.

The figures below show the enormous advantage of platinum-bismuth oxide systems over platinum-only systems, since only the former selectively produce DHA, even at room temperature and only when stirred in contact with atmospheric oxygen.

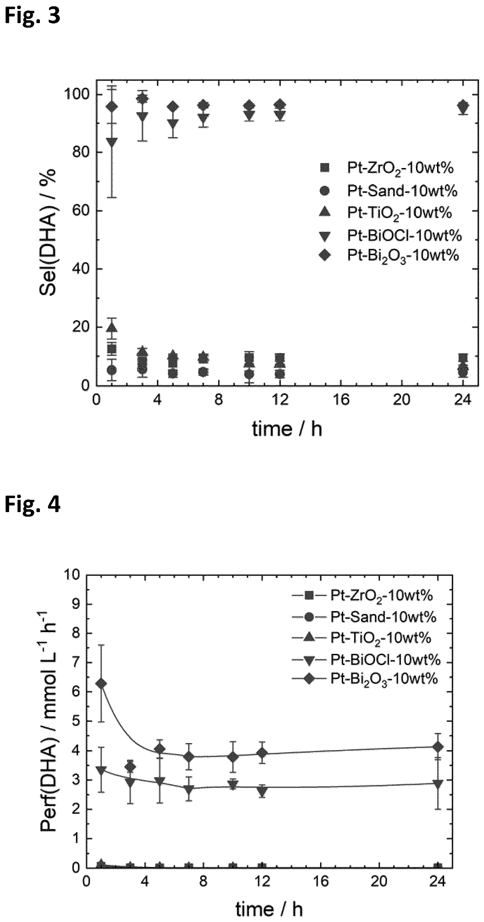

Fig. 2 shows the linear increase in DHA with time, Fig. 3 the constant selectivity of the desired product and Fig. 4 the corresponding constant performance (concentration of DHA per time) at given reaction conditions: Batch reactor V(total) = 20 mL, c(Gly)t=0 = 10 vol%, m(cat) = 320 mg, pH = 1.8 (adjusted with HCl), T = 23 °C, stirring speed 750 rpm, in constant contact with atmospheric oxygen.

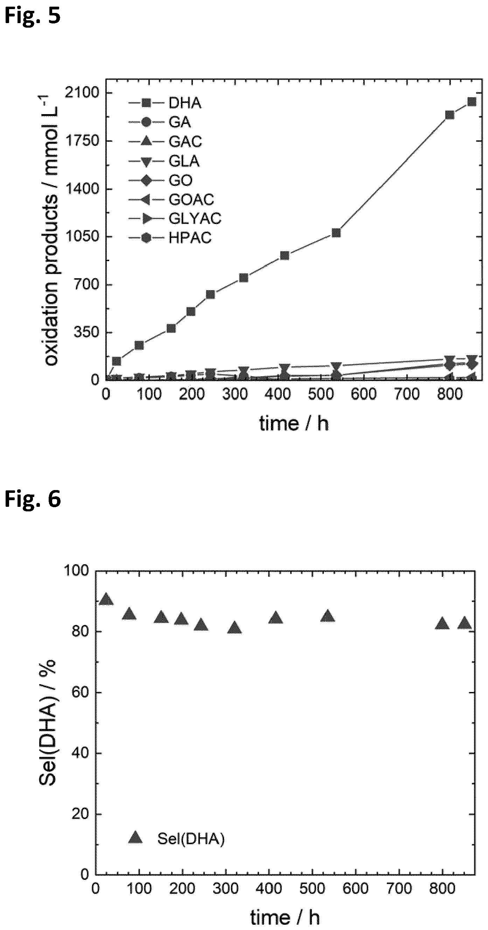

Figs. 5-7 demonstrate the high stability of platinum-bismuth catalysts using a 5-fold upscaled long-term experiment under the following reaction conditions: batch reactor V(total) = 100 mL, c(Gly)t=0 =50 vol.-%, m(Pt - Bi₂O₃ - 10 wt. %) = 320 mg, pH = 1.8 (adjusted with HCl), T = 23 °C, stirring speed 750 rpm, in constant contact with atmospheric oxygen, so that even after almost 1000 h the 90% of the initial selectivity (Fig. 6) is retained.

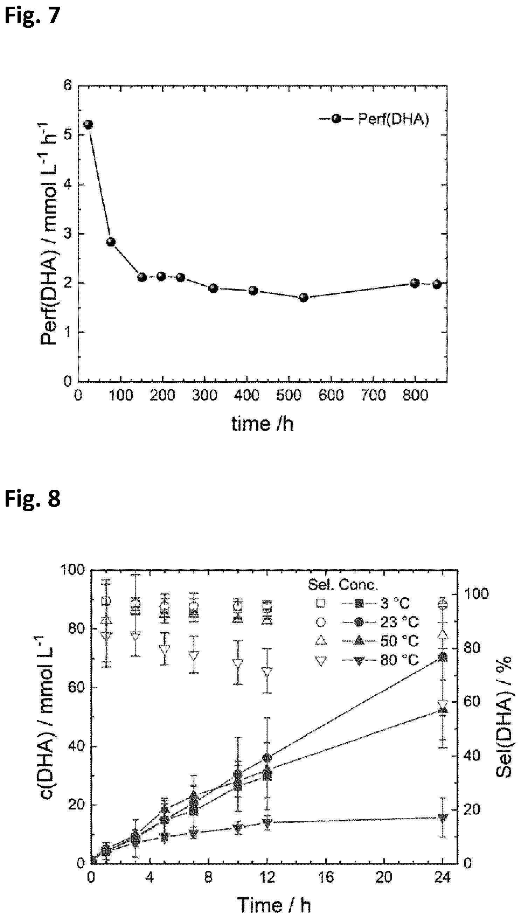

Fig. 8 demonstrates the achieved high selectivities which normally are very unusual for thermal catalytic glycerol conversion to DHA, which the inventors - without being bound to theory - ascribe to the fact that the method can be carried out at room temperature. As shown in figure 8, the lower temperatures are clearly beneficial for high selectivity. In figure 8 the Selectivity (open symbols) and DHA concentration (full symbols) for Pt-Bi₂O₃-10wt% catalyst is displayed at at different reaction temperatures. The applied reaction conditions are: V(total) = 20 mL, c(Gly)_t=0 = 10% vol, m(cat) =320 mg, pH=1.8 (set with HCl), stirring speed 750 rpm, under air. Since high conversion rates could be achieved even at room temperature, the inventors realized for the first time that a room temperature process leads to unprecedentedly high selectivity.

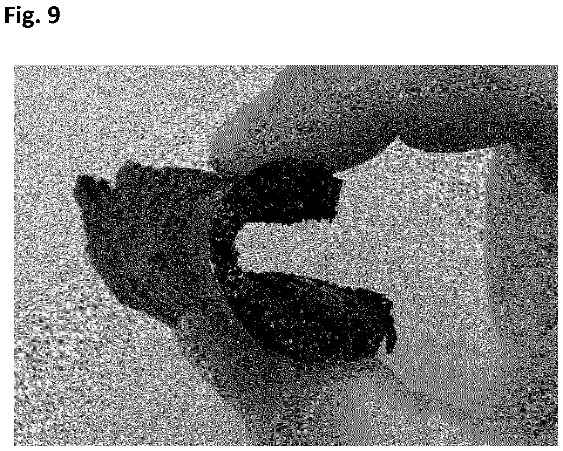

Moreover, as the process can be easily scaled up and operated for more than 850 hours without compromising the selectivity (selectivity after >850 hours was >82 %, the concentrations (ca. 2 mol/L DHA in water/glycerol mixtures - higher concentrations are easily achievable upon evaporation of water) and amounts of DHA can be synthesized that are enough to guarantee formation of biodegradable polymers upon simple addition of conventional reagents, as shown in figure 9, showing an optical image of a biodegradable polymer obtained by the following procedure using an upscaled reactor: DHA formation reaction conditions: V(total) = 200 mL, c(Gly)_t=0 = 10% vol, m(cat) = 3.2 g, pH=1.8 (set with HCl), stirring speed 750 rpm (+/- 20) , under air. Polymerisation: polymerisation can be carried out via Maillard reaction using lysine (see: J.A. Rufián-Henares, S. Pastoriza, Maillard Reaction, Editor(s): Benjamin Caballero, Paul M. Finglas, Fidel Toldrá, Encyclopedia of Food and Health, Academic Press, 2016, Pages 593-600, https://doi.org/10.1016/B978-0-12-384947-2.00435-9); Example: DHA formation reaction was stopped after 851 hours (at conversion of glycerol of ca. 30 %), the catalyst was filtered out, water was evaporated to 50 % of original volume, the mixture was heated to 85 °C, then lysine was added (1:1 molar ratio DHA : lysine) and kept without stirring at 85 °C for 5 minutes, leading to the formation of the polymer. This experiment showcases the viability of the disclosed process to produce DHA in amounts that are directly technologically applicable.

## Claims

1. Method for the preparation of 1,3-dihydroxyacetone (DHA) comprising the selective catalytic conversion of glycerol in the presence of
a) a catalytic amount of a heterogeneous catalyst comprising or consisting of a composite of a metal cluster and/or a metal oxide cluster and a metal chalcogenide semiconductor powder, an antimony-containing powder and/or a lead-containing powder or pluralities of the aforementioned compounds and
b) one or more oxidizing agents,
**characterized in that** the catalytic reaction is carried out in the absence of UV and visible light and the reaction is not conducted electrochemically.

2. Method according to claim 1, **characterized in that** in the catalyst, the metal or metal oxide is selected from Pt, Au, Ag, Ru, Rh, Pd, Cu, Co, Mn and Ni, and is preferably Pt.

3. Method according to one of the preceding claims, **characterized in that** in the catalyst, the metal chalcogenide semiconductor powder is selected from semiconductor metal oxides, semiconductor metal sulfides and semiconductor metal tellurides, preferably from bismuth-containing oxide semiconductor powders, especially Bi₂O₃, Bi₂S₃, Bi₂Te₃, Bi₂Se₃, BiOCI, BiOBr, BiOI, BiVO₄ and Bi₂MoO₆, and is preferably Bi₂O₃.

4. Method according to one of the preceding claims, **characterized in that** the amount of metal or metal oxide in the catalyst is from 0.01 to 90 % by weight, preferably from 1 to 10% by weight, based on the total weight of the catalyst.

5. Method according to one of the preceding claims, **characterized in that** the amount of catalyst is from 0.01 to 100 g per kg, preferably from 0.5 to 5 g per kg of glycerol to be converted.

6. Method according to one of the preceding claims, **characterized in that** in the heterogeneous catalyst the composite consists of bismuth-containing semiconductor powder coated with metal or metal oxide clusters.

7. Method according to one of the preceding claims, **characterized in that** the catalyst is obtainable by photoreductive deposition of one or more metal precursor compounds on the bismuth-containing semiconductor powder.

8. Method according to one of the preceding claims, **characterized in that** the Bi₂O₃ coated with Pt.

9. Method according to any of the preceding claims, **characterized in that** the oxidizing agents are selected from water, air, hydrogen peroxide, and are preferably water and/or air.

10. Method according to one of the preceding claims, **characterized in that** water is used as the oxidizing agent and hydrogen is obtained as a by-product, and preferably a weight ratio of glycerol to water of 1:100 to 10:1 is used.

11. Method according to one of the preceding claims, **characterized in that** the reaction is carried out at temperatures between 10 and 90°C, preferably between 15 and 60°C, particularly preferably between 20 and 40°C.

12. Method according to one of the preceding claims, **characterized in that** the reaction is carried out continuously or in batch mode, the reaction time preferably being at least 1 h.
